# EUROPEAN PATENT SPECIFICATION

(11) **EP 2 087 894 B1**
(45) Date of publication and mention of the grant of the patent: **14.08.2019**
(21) Application number: 09152528.7
(22) Date of filing: 11.02.2009
(51) Int. Cl.: A61P 17/18, A61K 31/7034, A61K 31/05, A61K 45/06

(54) **FORMULATIONS COMPRISING PICEID ABLE TO PREVENT AND INHIBIT LIPID PEROXIDATION**
FORMULIERUNGEN, DIE PICEID UMFASSEN, DIE LIPIDPEROXIDATION VERHINDERN UND INHIBIEREN KÖNNEN
FORMULES COMPRENANT DU PICEID POUVANT EMPÊCHER ET INHIBER LA PÉROXYDATION DE LIPIDE

(30) Priority: 11.02.2008 IT FI20080019
(43) Date of publication of application: 12.08.2009
(73) Proprietor: Glures S.R.L., 30170 Mestre - Venezia (IT)
(72) Inventor: Stevanato, Roberto, 30174, MESTRE (IT); Ravagnan, Giampietro, 00164, ROMA (IT); Momo, Federico, 30125, VENEZIA (IT); Fabris, Sabrina, 30125, VENEZIA (IT)
(74) Representative: Gervasi, Gemma

(56) References cited:
- WO-A-00/44921
- WO-A-2007/020673
- US-A1- 2007 270 496
- US-B1- 6 414 037
- DATABASE WPI Week 200754 Thomson Scientific, London, GB; AN 2007-546252 XP002530443 & CN 1 927 212 A (XU J) 14 March 2007 (2007-03-14)
- JANG D-S ET AL: "Inhibitory effects of resveratrol analogs on unopsonized zymosan-induced oxygen radical production" BIOCHEMICAL PHARMACOLOGY, PERGAMON, OXFORD, GB, vol. 57, no. 6, 15 March 1999 (1999-03-15), pages 705-712, XP002322563 ISSN: 0006-2952
- WAFFO-TEGUO PIERRE ET AL: "Potential cancer-chemopreventive activities of wine stilbenoids and flavans extracted from grape (Vitis vinifera) cell cultures" NUTRITION AND CANCER, vol. 40, no. 2, 2001, pages 173-179, XP009065742 ISSN: 0163-5581
- RYU S Y ET AL: "Antitumor activity of some phenolic components in plants" ARCHIVES OF PHARMACAL RESEARCH, NATL. FISHERIES UNIVERSITY, PUSAN, KR, vol. 17, no. 1, 1 January 1994 (1994-01-01), pages 42-44, XP002968965 ISSN: 0253-6269

## Description

### Field of the invention

The present invention relates to the field of formulations containing piceid possibly in combination with resveratrol.

### State of the art

Piceid and resveratrol, pertaining to the stilbene class, are well-known products.

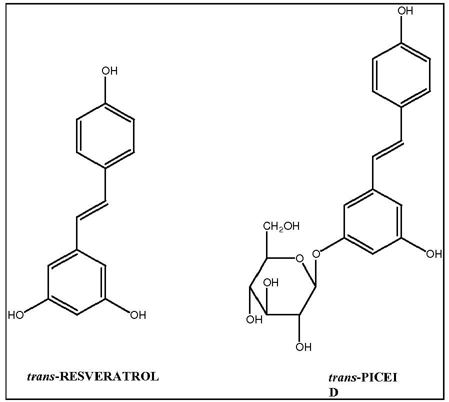

For example European patent applications EP 1292320 and EP 1292319 describe the products and processes for extracting pharmacologically active products from spermatophyte plants, including resveratrol and piceid; also reported are the immunomodulatory and cytotoxic properties of these products, useful for the treatment of tumours. In the case of resveratrol, several studies have been conducted on its antioxidant abilities and consequent beneficial properties owing to its inhibitory effects on cancer formation and propagation, its cardioprotective action due to the inhibition of low density lipoprotein (LDL) oxidation and of platelet aggregation, its anti-inflammatory activity and its preventive effects on Alzheimer's disease and dementia.

There is however very little information on piceid, the glucosylated form of resveratrol which is found to be present in nature in a quantity approximately seven times that of resveratrol itself and is hypothesized to be the most widespread stilbene form in nature. In particular, no significant comments have hitherto been published on its antioxidant characteristics and its ability to prevent and inhibit lipid peroxidation which, as is known, is the cause of many important pathologies.

On the other hand, as aforementioned, resveratrol has been shown to exhibit innumerable beneficial properties in the treatment of important pathologies on account of its antioxidant properties. By demonstrating that piceid also presents similar, if not actually superior properties, the potential for their use is considerably extended, either singly or in combination in pharmaceutical, cosmetic and skin protective formulations, also taking into account the great abundance of piceid in natural matrices.

### Detailed description of the invention

It has been discovered, and represents an aspect of the present invention, that piceid, like resveratrol in the two forms *cis* and *trans*, exhibits antioxidant characteristics of interest and that the two stilbenes, either singly or in combination, are able to act particularly effectively in preventing and inhibiting lipid peroxidation. It is known that lipid peroxidation is a process which damages animal and plant cells. Lipids rich in polyunsaturated fatty acids, which are mainly present in cell membranes, easily undergo hydrogen removal in the presence of free radicals, they themselves being transformed into radicals which react rapidly with oxygen to form peroxyl radicals. These latter remove one atom of hydrogen from other lipid molecules to create further radicals, in this manner giving rise to an autooxidation cycle with the systematic destruction of lipids and serious damage to membranes, compromising actual cell survival.

In the light of the aforesaid, there appears to be no doubt of the benefit of using those natural i.e. not synthesized, components which are extensively widespread in nature and already present in the food ingredients of the Mediterranean diet (fruit, vegetables, wine, etc), as effective antioxidants in the formulation of pharmaceutical, cosmetic and skin protective products, as well as for preserving foods from chemical oxidation and photooxidation.

The antioxidant activity of *trans*-resveratrol and *trans*-piceid has been verified as follows:
a) by studying their inhibitory action on the peroxidation of linoleic acid in SDS (sodium dodecyl sulphate) micelles and unilamellar liposomes of DPPC (dipalmitoyl L-alpha-phosphatidylcholine, C16:0);
b) by measuring their ability to neutralize different free radicals, such as DPPH (2,2'-diphenyl-1-picrylhydrazyl) and TEMPO (2,2,6,6-tetramethyl-1-piperidinyloxy) and radical initiators ABIP (2,2'-azobis[2-(2-imidazolin-2-yl)propane), AAPH (2,2'-azobis(2-amidinopropane)), ABCPA (4,4'azobis(4-cyanopentanoic acid)), then comparing the data thus obtained with those of the two known antioxidants, vitamin E (alpha-tocopherol) and BHT (2,6-di-tert-butyl-4-methyl-phenol).

The effect of the antioxidants on lipid peroxidation was verified by measuring the quantity of oxygen consumed at 37ºC as a consequence of the radical reaction thermally initiated by ABIP. Under these conditions a steady decline in oxygen is observed which drastically decreases after addition of antioxidant.

Both piceid and resveratrol show a steady decrease in oxygen consumption greater than that of vitamin E and BHT (apart from a brief initial period of strong inhibition exhibited by the latter) but with a more prolonged effect over time.

With DHHP, the radical neutralizing activity was instead followed spectrophotometrically by measuring the initial rate (Vo) of the disappearance of its characteristic band at 515 nm. The data collected show that the Vo values of resveratrol and piceid are comparable to those of BHT (although lower than those of vitamin E) which confirms the good neutralizing capability of the two products examined. It was also required to verify whether the two products examined are able to react with the free radical initiators before the latter activate lipid peroxidation in the membrane.

In contrast to the case of BHT (vitamin E was not tested due to its insolubility in water), resveratrol and piceid were found to react with all the free radicals and radical initiators under examination.

This peculiar and extraordinary reactivity suggests that such compounds could be able to block since the beginning the radicals chain and therefore presents themselves as potential agents in the preventive inhibition of lipids peroxidation.

The above said action is important also for the formation of the epoxides which, as it is known, are normally very reactive and binds to nucleophile groups of proteins and DNA starting dangerous pathologies.

Cholesterol is one of the most important component of animal plasma cell membranes to which it imparts the necessary rigidity because of its structural characteristics. Moreover cholesterol is present in the lipoproteins of blood plasma although in the form of derivatives.

It was demonstrated that in the skin irradiated with UV cholesterol undergoes oxidation with the formation of the corresponding epoxide (5α,6α-epoxy-colestan-3β-οl) through a radical mechanism. The cholesterol epoxide is considered the probable cause of the carcinogenic of the UV and as a direct mutagenic agent (see for example Sevanian et. Al Proc. Natl Acad Sci USA 81/13, 4198 - 4202 (1984) and Morrin R.J. J. Clin. Lab. Anal. 5/3, 219 - 225 (1991)).

The formation of cholesterol epoxide and therefore the risk of important tumours can be prevented by the action of piceid and resveratrol in derma-protective formulations thanks to their capacity in blocking the action of free radicals.

It was reported that the epoxidation of 17-β-estradiole and of the estrone could be the principal factor in the breast tumour. Tamoxifene, an anti-estrogen used for the treatment of breast tumour, shows also effective properties in preventinve the tumour. It is considered that the preventive action is due to tamoxifene ability in preventing estradiole epoxidation through a competitive mechanism.

On the other hand are well known the side effects of tamoxifene that can be considred in its turn as a cancer initiator because of its capacity, once metabolically activated, to bind to DNA inducing, for example, endometrial tumours.

The combined action of piceid and resveratrol in the inhibition of the estradiole epoxide formation can therefore be effective in curing and preventing breast tumour reducing the risk of secondary reaction on DNA and therefore the rise of new tumours (see Yu, F.L. Asia Pac. J. clin. Nutr. 11/7, 460 - 66 (2002) and Cancer Detect. Prev. 26, 370-5 (2002)).

In particular, the initial rate of piceid consumption is similar to that of ABIP decomposition, while that of resveratrol is five times greater. Quantitative evaluations have shown that piceid is more effective than resveratrol because the latter stilbene is able to undergo reaction with its radical form.

As the antioxidant efficacy of both products is related to their partitioning between the water and lipid phase and to their position in the membrane bilayer, an investigation was carried out on the interactions of resveratrol and piceid in multilamellar liposomes of saturated L-alpha-phosphatidylcholine (PC) of various chain lengths (dimyristoyl, DMPC; dipalmitoyl, DPPC; distearoyl, DSPC) to determine: a) partition coefficients of the stilbenes between liposomes and water; b) modification of lipid bilayer organization; c) their position in the membrane using differential calorimetric analysis (DSC) and spin labelling (EPR).

It can be observed from partition coefficient measurements that, as envisaged, piceid is less hydrophobic than resveratrol due to the presence of the very hydrophilic glucosidic residue in the molecule. In spite of this, both molecules are sufficiently lipophilic to partition themselves almost exclusively in the lipid phase. The DSC profiles of the gel-to-fluid state transition of DMPC (dimyristoylphosphatidylcholine, of 14 C atoms), DPPC (dipalmitoylphosphatidylcholine, of 16 C atoms) and DSPC (distearoylphosphatidylcholine, of 18 C atoms) multilamellar liposomes at increasing resveratrol and piceid concentrations, are broadened and shifted towards lower temperatures for increasing stilbene concentrations, which demonstrates that both the size and the packing of the cooperative units undergoing the transition are modified by the antioxidants, hence weakening the lipid bilayers.

From the EPR spectra of stearic acids spin labelled at carbon positions 5, 7, 10, 12 and 16 (n-SASL) and incorporated into DPPC liposomes, it can be seen that both resveratrol and piceid are located in the hydrophobic region of the bilayer although piceid is closer to the hydrophilic region. Moreover, the hydroxyl group of the two molecules, being particularly reactive to free radicals, is very close to the two double bonds of the polyunsaturated fatty acids (PUFA), whereas that of vitamin E is more displaced towards the polar surface, and that of BHT, in contrast, is located more deeply.

It follows that because of simple proximity reasons, piceid and resveratrol can react more rapidly and effectively than vitamin E and BHT with the peroxyl radical which forms following lipid peroxidation.

For the following reasons, we can therefore conclude that resveratrol and piceid are excellent antioxidants:
- they are sufficiently hydrophobic to partition themselves mainly in the membrane lipid bilayer;
- they prevent lipid peroxidation by reacting with a wide spectrum of radicals and radical initiators;
- they inhibit lipid peroxidation by reacting effectively with peroxyl radicals which form in the peroxidation propagation phase;
- they are located within the membrane in such as manner that their hydroxyl group, being particularly reactive to free radicals, is found in the immediate vicinity of the double bonds of the polyunsaturated fatty acid (PUFA) residues, these more easily undergoing lipid peroxidation.

Products which can hence be formulated, according to the invention, are:
- solutions and emulsions such as additives for the natural preservation of products (foods, drugs, cosmetics, sun-protection products, etc.) which are perishable due to chemical oxidation or photooxidation, and can either substitute or supplement other synthetic preservatives;
- food supplements with the aim of increasing the level of antioxidant substances in the body, particularly in cases of deficiency of consumption of those foods (wine, fruit and vegetables) in which these substances are naturally present;
- skin protective oils, emulsions, suspensions, creams and ointments for preventing oxidative and photooxidative damage caused by lengthy exposure to the sun, whose ultraviolet radiation is known to be the cause of free radical formation;
- cosmetic preparations to prevent possible damage due to other components present in the formulation;
- anti-inflammatory drugs for oral, local and topical use.

Compositions containing the active principles can be prepared by following known techniques in the pharmacopeia for producing products of this type, at concentrations that depend on the benefits required and the means of administration, as well as on subjective parameters such as weight and age of the person, and any pathologies in progress and/or the specific sensitivity to the stilbenes under discussion. Considering the quantities of these two substances present in widely consumed natural foods (Phytochemistry, 37/2, 571, 1994; J. Agric. Food. Chem. 47, 1533, 1999; Meth. Enz. 299, 184, 1999), it is expedient that in food supplement or additive formulations the piceid and resveratrol quantities, in that ratio of the two *trans*/*cis* isomeric forms generally present in nature in which the *cis* isomer is decidedly lower than the *trans* isomer (J.Agric.Food.Chem. 47, 1533, 1999), should be between 0.0001 and 1 milligram per gram or millilitre of product in the case of oils, creams and ointments, while for oral consumption the maximum quantity should not exceed 0.1 milligrams per gram of product.

Higher dosages can be consumed but only under strict medical control, taking into consideration the information given in patents EP 1292319B1 and EP1292320B1 relating to the cytotoxicity of said compounds and in particular of cis-resveratrol.

## Claims

1. Antioxidant compositions containing piceid, possibly in combination with resveratrol for use in the prevention of oxidative and photooxidative damage of the skin caused by lengthy exposure to the sun in the form of skin protective oils, suspensions, creams, ointment, solutions and/or emulsions.

2. Compositions for use according to claim 1 wherein the quantity of active principle is comprised between 0.0001 and 1 milligram per gram or millilitre of product.

3. Compositions for use according to claim 2 wherein the quantity of active principle is less than 0.1 milligrams per gram of product.

## Patentansprüche

1. Piceidhaltige antioxidative Zusammensetzungen, gegebenenfalls in Kombination mit Resveratrol zur Verwendung bei der Vorbeugung von oxidativer und photooxidativer Schädigung der Haut, verursacht durch längere Sonneneinstrahlung in Form von Hautschutz-Ölen, - Suspensionen, -Cremes, -Salbe, -Lösungen und/oder -Emulsionen.

2. Zusammensetzungen zur Verwendung nach Anspruch 1, wobei die Menge des Wirkstoffs aus zwischen 0,0001 und 1 Milligramm pro Gramm oder Milliliter Produkt besteht.

3. Zusammensetzungen zur Verwendung nach Anspruch 2, wobei die Menge des Wirkstoffs weniger als 0,1 Milligramm pro Gramm Produkt beträgt.

## Revendications

1. Compositions antioxydantes contenant le piceid, éventuellement en association avec le resvératrol, à utiliser dans la prévention des lésions oxydatives et photo-oxydatives de la peau causées par une exposition prolongée au soleil, sous forme d'huiles, de suspensions, de crèmes, de pommade, de solutions et/ou d'émulsions protectrices de la peau.

2. Compositions à utiliser selon la revendication 1, dans lesquelles la quantité de principe actif est comprise entre 0,0001 et 1 milligramme par gramme ou millilitre de produit.

3. Compositions à utiliser selon la revendication 2, dans lesquelles la quantité de principe actif est inférieure à 0,1 milligramme par gramme de produit.
